(19) Europäisches Patentamt

European Patent Office

Office européen des brevets

(11) Veröffentlichungsnummer: **0 307 528 B1**

(12) **EUROPÄISCHE PATENTSCHRIFT**

(45) Veröffentlichungstag der Patentschrift: **15.07.92**

(21) Anmeldenummer: **87730160.6**

(22) Anmeldetag: **01.12.87**

(51) Int. Cl.⁵: **A61B 1/22**

──────────────────

(54) **Nasenspekulum.**

──────────────────

(30) Priorität: **08.09.87 DE 8712300 U**
**17.09.87 DE 8712654 U**

(43) Veröffentlichungstag der Anmeldung:
**22.03.89 Patentblatt 89/12**

(45) Bekanntmachung des Hinweises auf die
Patenterteilung:
**15.07.92 Patentblatt 92/29**

(84) Benannte Vertragsstaaten:
**AT BE CH DE ES FR GB GR IT LI NL SE**

(56) Entgegenhaltungen:
**FR-A- 2 316 855**
**FR-A- 2 478 458**
**US-A- 2 592 190**
**US-A- 3 664 330**
**US-A- 4 314 551**

**Anästh. Intensivther. Notfallmed. 19 (1984),
Seiten 310,311, Georg Thieme Verlag, Stuttgart, New York**

(73) Patentinhaber: **Effner GmbH
Alt-Lankwitz 102
W-1000 Berlin 46(DE)**

(72) Erfinder: **Anapliotis, Emmmanuel
Kiebitzweg 5
W-1000 Berlin 33(DE)**

(74) Vertreter: **Christiansen, Henning, Dipl.-Ing. et
al
Patentanwalt CHRISTIANSEN Pacelliallee
43/45
W-1000 Berlin 33(DE)**

## Beschreibung

Die Erfindung betrifft ein Nasenspekulum gemäß Anspruchs 1.

Es ist bekannt, für Untersuchungszwecke oder zum Einführen eines Beatmungs- oder Trachealtubus zur künstlichen Beatmung oder zur Intubationsnarkose ein Nasenspekulum zu verwenden, dessen Branchen in die Körperöffnung eines Patienten eingeführt und gegen die Wirkung einer Rückstellfeder mittels mit den Branchen verbundenen Griffhebeln auseinanderbewegt werden, so daß ein Kanal zur Einsichtnahme in die betreffende Körperhöhle des Patienten für einen behandelnden Arzt geschaffen wird. Der so geschaffene Kanal bzw. lichte Raum kann mittels eines Spiegelreflektors oder einer anderen Lichtquelle beleuchtet werden, um das Anbringen eines Tubus oder die Erkennbarkeit der untersuchten Korperhöhle für Diagnosezwecke zu erleichtern.

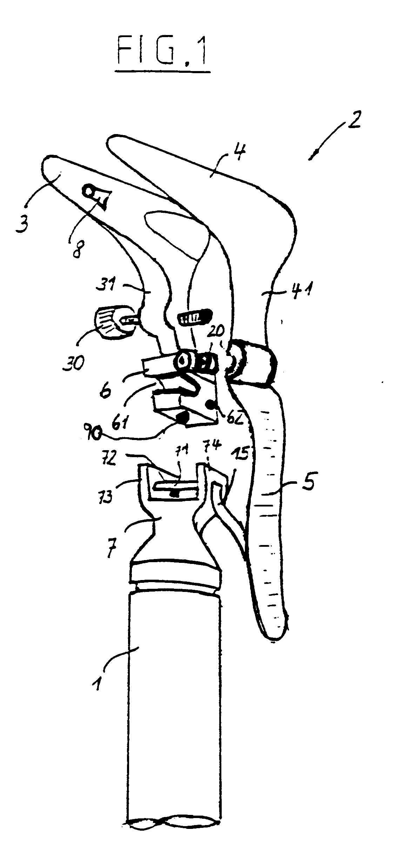

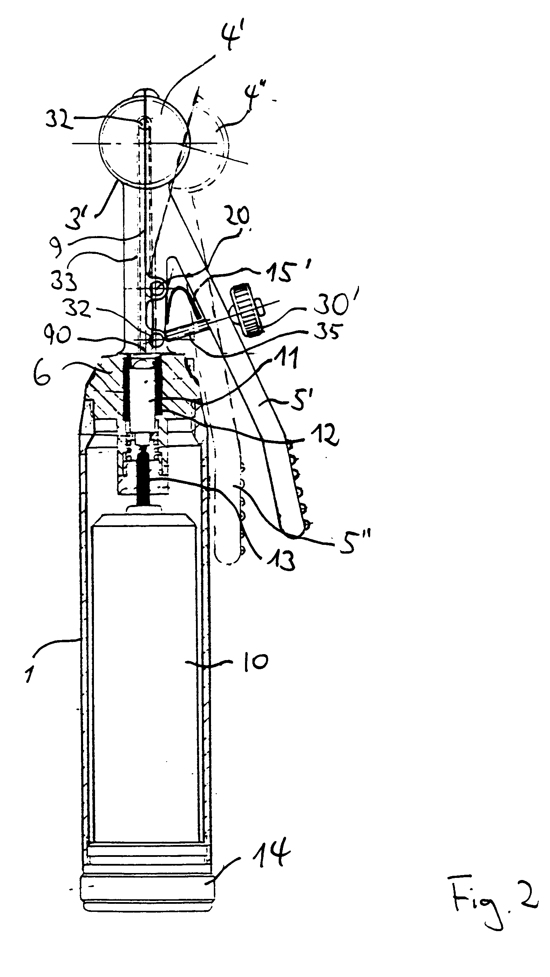

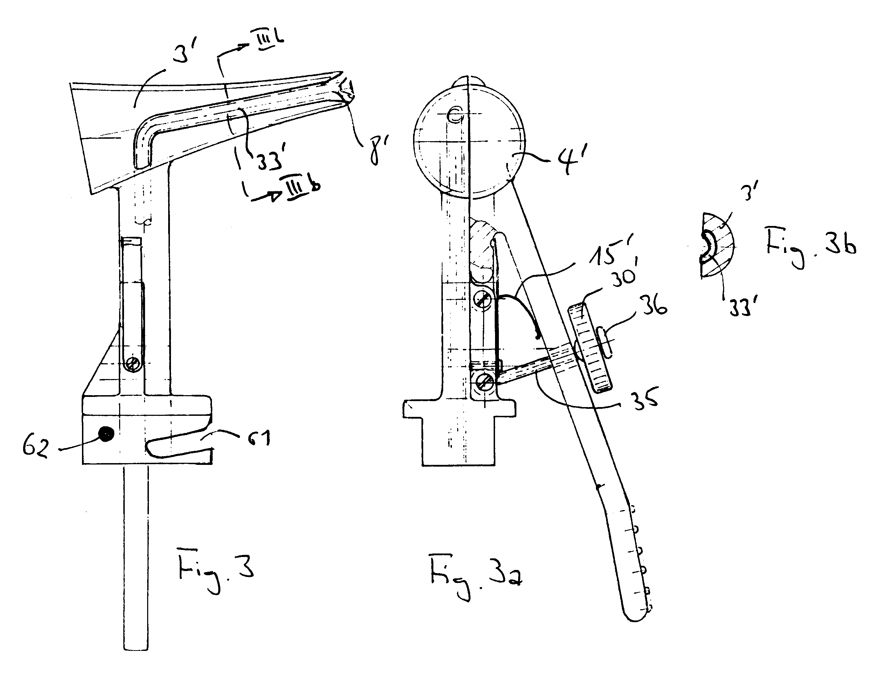

Ein entsprechendes Nasenspekulum ist aus Anäsh. Intensivther. Notfallmed. 19 (1984), Seite 311, Abb. 1 bekannt.

Hierbei ist jedoch nachteilig, daß Batterieteil und Nasenspekulum über eine flexible Leitung miteinander verbunden sind und somit keine einhandige Bedienung möglich ist. Einfache Bedienbarkeit ist jedoch bei medizinischen Instrumenten, insbesondere während eines chirurgischen Eingriffs, eine wesentliche Voraussetzung.

Aus der DE-C-27 38 202 ist ferner ein Laryngoskop für Kehlkopfuntersuchungen bekannt, das einen Spatel aufweist, in dem ein Lichtleitfaserbündel verlegt ist und in einer distalen Lichtaustrittsfläche endet, aus der Licht zur Beleuchtung eines zu betrachtenden Objektes austritt. Am anderen Ende ist das Lichtleitfaserbündel durch einen Kontaktfuß des Spatels geführt, der in einen auf einer Stirnseite eines als Handgriff ausgebildeten Gehäuses rastend einsetzbar ist. In dem Aufsatz ist eine mit dem Ende des Lichtleitfaserbündels gekoppelte Glühlampe angeordnet, die von einem in dem Handgriff angeordneten Batterieeinsatz gespeist wird. Durch die lösbare Verbindung zwischen Spatel bzw. Kontaktfuß und dem als Handgriff ausgebildeten Gehäuse bzw. dem an der Stirnseite des Gehäuses angebrachten Aufsatz können Spatel unterschiedlicher Größe in Verbindung mit einem Batterieeinsatz verwendet werden. Darüberhinaus ermöglicht die lösbare Verbindung ein leichtes Auswechseln der im Aufsatz angeordneten Glühlampe, wenn Spatel und Gehäuse voneinander getrennt sind. Hierbei besteht jedoch nicht die Möglichkeit, eine Spreizung durchzuführen. Auch fehlen selbstverständlich diesbezügliche Betätigungselemente.

Ferner ist ein Nasenspekulum ist aus Anäsh. Intensivther. Notfallmed. 19 (1984), Seite 311, Abb. 2 bekannt, bei dem ohne Lichtleiterführung in die Branchen hinein als Aufsatz für ein Batteriegehäuse ein Nasenspreizer vorgesehen ist. Die Beleuchtung wird durch Reflexion an den Brancheninnenseiten erzielt. Der Betätigungsgriff der beweglichen Branche ist in Branchenrichtung angeordnet.

Weiterhin ist aus der US-A-4 314 551 ein Laryngoskop zum Einführen eines Trachealtubus zur Intubationsnarkose bekannt, bei dem zwei schnabelförmige Branchen mit einem Griffhebel gegen die Wirkung einer Rückstellfeder auseinanderbewegbar sind. An einer Innenfläche der nicht beweglichen Branche ist eine Lichtquelle in Form einer Glühlampe angebracht welche über Kabel mit einer im Handgriff angeordneten Batterie als Spannungsquelle verbunden ist. Nachteilig ist hierbei die an der Branche angeordnete Glühlampe, da sie nur durch ein Herausnehmen des Laryngoskops während eines Eingriffs ausgewechselt werden kann. Weiterhin ist der Griffhebel in unvorteilhafter Weise zwischen dem Patienten und dem Handgriff angeordnet wodurch der ausführende Arzt in seinen Bewegungen eingeschränkt ist und der Patient das Instrument als einengend empfinden kann. Die untere bewegliche Branche des Laryngoskops wird in Richtung des Handgriffs nach unten aufgespreizt. Der Einsatz eines solchen Laryngoskops als Nasenspekulum wäre sogar nach Anpassung der Branchenform an die der Nase nicht geeignet, da der Handgriff des Instruments in einer wesentlich schwieriger einzuhaltenden waagerechten Lage gehalten und betätigt werden müßte, um das Instrument in die Nase in die erwünschte Richtung einführen zu können.

Aufgabe der vorliegenden Erfindung ist es, ein Nasenspekulum der eingangs genannten Art zu schaffen, das eine optimale Ausleuchtung der zu betrachtenden Körperhöhle eines Patienten auch bei Verwendung eines Nasenspekulums mit schnabelförmigen Branchen gewährleistet und dabei unkompliziert handhabbar ist.

Diese Aufgabe wird durch das Nasenspekulum gemäß Anspruchs 1 gelöst.

Der Erfindung liegt die Erkenntnis zugrunde, daß die Anordnung einer distalen Beleuchtungseinrichtung an mindestens einer der Branchen über eine Glasfaserleitung verbunden mit einem unmittelbar in fester Arretierung anschließenden, unabhängige Energiequellen und die Lichtquelle enthaltenden Handgriff eine Beleuchtung der zu betrachtenden Körperhöhlen unabhängig von der Anordnung einer äußeren Lichtquelle bzw. der Stellung eines Spiegelreflektors ermöglicht.

Die Spannungsquelle besteht aus einer in einem Gehäuse angeordneten Batterie oder einem Akkumulator, wobei das Gehäuse einen stirnseitigen Aufsatz aufweist, in den ein Kontaktfuß der mit der distalen Beleuchtungseinrichtung versehenen Branche abnehmbar einrastbar ist. Auf diese Weise lassen sich Batteriehandgriffe einsetzen, die bereits bei Laryngoskopen der eingangs genannten Art eingesetzt werden, so daß ein wirtschaftlicher Einsatz von Geräten geschaffen wird, mit denen die zu betrachtenden Körperhöhlen eines Patienten unmittelbar ausgeleuchtet werden können.

Dadurch wird sichergestellt, daß insbesondere bei einem Einsatz des Nasenspekulums in Notfällen wie zur Einführung eines Beatmungstubus eine Reserve-Spannungsquelle in Form eines zweiten mit einem Batterieeinsatz versehenen Handgriffgehäuses zur Verfügung steht und daß die Verwendung von Nasenspekula mit unterschiedlich langen Branchen unter Einsatz eines Batteriehandgriffes bzw. eines weiteren Reserve-Batteriehandgriffes ermöglicht wird.

Hierbei wird durch die Verwendung eines Anschlußelements (Schnittstelle) am Batteriehandgriff entsprechend demjenigen eines Laryngoskops sichergestellt, daß die Handgriffe - und damit die Energiequellen - von in derselben Operationsphase verwendeten Instrumenten austauschbar sind. Damit vereinfacht sich einerseits die Lagerhaltung und andererseits kann auch im Notfall der Handgriff des einen Instruments durch denjenigen des anderen ersetzt werden, um im Falle der Erschöpfung einer Energiequelle umgehend Ersatz zu haben, da die Schnellwechseltechnik der Schnittstelle einen unverzüglichen Austausch ohne besondere Aufmerksamkeitserfordernisse erlaubt.

Bei der Erfindung ist der Betätigungsgriff der beweglichen Branche derart angeordnet, daß er seitlich zugänglich ist, so daß durch den Griff keinerlei Behinderung der Beweglichkeit auf der Anwendungsseite in Branchenrichtung entsteht. Durch einen seitlich liegenden Betätigungsgriff ist auch eine gleichermaßen gute Bedienbarkeit für Links- und Rechtshänder gegeben.

Dadurch daß, - entsprechend einer anderen vorteilhaften Weiterbildung der Betätigungsgriff bei Lagerung innerhalb der fortgesetzten Querschnittskontour des Batterieteils bei extremer Betätigung der seitlichen Kontourlinie folgt und/oder an dieser anliegt, ist ein maximaler Betätigungsweg erzielbar, wobei auch in unbetätigtem Zustand der Griff einen minimalen zusätzlichen Raum einnimmt.

Dabei ist bevorzugt die bewegliche Branche mittels eines Gelenkes mit der den Kontaktfuß aufweisenden Branche verbunden, wobei der mit der beweglichen Branche verbundene Griffhebel mittels der zwischen Griffhebel und Kontaktfuß angeordneten Rückstellfeder in die gespreizte Stellung geführt wird.

Damit wird ein Nasenspekulum geschaffen, das in seiner äußeren Form und Handhabung dem eines konventionellen unbeleuchteten Nasenspekulums gleicht, so daß der Einsatz des erfindungsgemäßen Nasenspekulums keine Umgewöhnung des behandelnden Arztes erforderlich macht.

In einer alternativen Ausführungsform ist die bewegliche Branche mit einer Schubstange verbunden, die durch eine oberhalb des Kontaktfußes angeordnete Bohrung in einem die Branche mit dem Kontaktfuß verbindenden Teil geführt ist, wobei die Schubstange an ihrem freien Ende einen Griffhebel aufweist, zwischen dem und dem Kontaktfuß eine Rückstellfeder angeordnet ist. Der Vorteil dieser alternativen Ausführungsform liegt darin, daß der Spreizvorgang durch Daumendruck gesteuert werden kann.

Zusätzlich kann eine Sperrklinkeneinrichtung nach Art einer Ratsche oder ein eine Rändelschraube aufweisende Gewindefeststellvorrichtung zwischen dem beweglichen und dem festen Griffhebel vorgesehen werden, so daß beim Zusammendrücken des Griffhebels und des Batteriegehäuses und damit beim Auseinanderdrücken der Branchen jede Zwischenstellung eingehalten wird.

In einer weiteren vorteilhaften Ausgestaltung der Erfindung ist auch die bewegliche Branche mit einer distalen Beleuchtungseinrichtung versehen, wobei die Beleuchtungseinrichtung ebenfalls aus einer Lichtaustrittsoptik, die über einen Lichtleiter mit der an die Spannungsquelle angeschlossenen Lichtquelle verbunden ist, besteht und die Verbindung zwischen der an der Innenfläche der beweglichen Branche angeordneten, distalen Beleuchtungseinrichtung und der Spannungsquelle über das die bewegliche Branche mit der feststehenden Branche verbindende Gelenk erfolgt.

Das Instrument ist in Einhandbedienung in günstiger Weise handhabbar, ohne daß Zuleitungen oder in Richtung des Patienten ragende Betätigungselemente die Anwendungsmöglichkeiten behindern.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 eine perspektivische Darstellung eines Nasenspekulums mit Beleuchtungseinrichtung mit Einzelteilen in Explosionsdarstellung;

Figur 2 eine teilweise geschnittene Draufsicht einer Ausführungsvariante des Nasenspekulums gemäß Figur 1;

Figur 3 ein Nasenspekulum als Aufsatz für ein Batterieteil in Seitenansicht;

Figur 3a die Ausführung gemäß Figur 3 in Draufsicht;

Figur 3b eine Schnittdarstellung eines Details der Ausführung gemäß Figur 3;

Figur 4 eine teilweise geschnittene Seitenansicht einer Variante des Nasenspekulums mit Beleuchtungseinrichtung und

Figur 5 einen Schnitt durch das Nasenspekulum gemäß Figur 4 entlang der Linie A - A.

Das in Figur 1 perspektivisch dargestellte Nasenspekulum mit Beleuchtungseinrichtung besteht aus einem als Handgriff ausgebildeten zylindrischen Gehäuse 1 mit einem an einer Stirnseite des Gehäuses 1 angeordneten Aufsatz 7, einer mit einem Kontaktfuß 6 verbundenen Branche 3 mit distaler Beleuchtungseinrichtung 8 und einer mit einem Griffhebel 5 verbundenen Branche 4, die gegenüber der Branche 3 beweglich und mit der Branche 3 über ein Gelenk 20 verbunden ist.

Die Verbindung der beiden Branchen 3, 4 des Nasenspekulums 2 erfolgt über das Gelenk 20 und eine in das Gelenk 20 eingesteckte Achse. Die mit dem Kontaktfuß 6 versehene Branche 3 enthält im Bereich des distalen Endes eine Beleuchtungsaustrittsöffnung 8.

Die Öffnungsabstand zwischen den beiden Branchen 3, 4 kann mittels einer Stellschraube 30 eingestellt werden, die durch einen die Branche 3 mit dem Kontaktfuß 6 verbindenden abgewinkelten Teil 31 geführt ist und deren Ende gegen einen die bewegliche Branche 4 mit dem Gelenk 20 verbindenden Schenkel 41 stößt, wobei der Öffnungswinkel zwischen beiden Branchen 3, 4 des Nasenspekulums 2 von der Länge des zwischen beiden Schenkeln 31, 41 befindlichen Teils der Stellschraube 30 abhängt.

Alternativ oder in Ergänzung hierzu kann zwischen dem Griffhebel 5 und dem Batteriegehäuse 1 eine Ratsche vorgesehen werden, die in Verbindung mit der Wirkung der Rückstellfeder 15 jede beliebige Stellung des Nasenspekulums bzw. der Branchen 3, 4 einhält. Durch ein geringes weiteres Zusammendrücken des Griffhebels 5 und des Batteriegehäuses 1 oder durch Umstellen eines Ratschenhebels wird die Verriegelung gelöst und die Branchen 3, 4 zusammengeführt.

Die zwischen dem Griffhebel 5 und dem Aufsatz 7 an der Stirnseite des Gehäuses 1 angeordnete Blattfeder 15 wirkt als Rückstellfeder, so daß in der Ruhestellung die Innenflächen der beiden Branchen 3, 4 aneinanderliegen bzw. einen Abstand zueinander aufweisen, der von der zwischen den Schenkeln 31, 41 befindlichen Länge der Stellschraube 30 abhängt. Die Blattfeder 15 kann bei diesem Ausführungsbeispiel wahlweise an der Innenseite des Griffhebels 5 befestigt und an der Außenfläche des gabelförmigen Aufsatzes 7 anliegen bzw. an der Außenfläche des gabelförmigen Aufsatzes 7 befestigt sein und in montiertem Zustand an der Innenfläche des Griffhebels 5 anliegen.

Der gabelförmige Aufsatz 7 weist zwei Seitenflächen 73, 74 auf, zwischen denen eine Achsstift 71 angebracht ist. Zusätzlich sind auf der Innenseite der Seitenflächen 73, 74 Nuten 72 vorgesehen, von denen in der perspektivischen Darstellung gemäß Figur 1 nur die in der einen Seitenfläche 73 vorgesehene Nut 72 zu erkennen ist. Der Aufsatz 7 bildet zusammen mit dem nachfolgend zu beschreibenden Kontaktfuß eine Schnellkupplung.

Der Kontaktfuß 6 weist eine Aussparung 61 und zwei an den Seitenflächen angebrachte Nocken 62 auf, deren Lage im montierten Zustand der der Nuten 72 in den Seitenflächen 73, 74 des gabelförmigen Aufsatzes 7 entspricht. An der Unterseite des Kontaktfußes 6 ist eine Kontaktfläche 90 vorgesehen, die einen optischen Kontakt mit einer entsprechenden Gegenkontaktfläche an der Oberseite des gabelförmigen Aufsatzes 7 herstellt.

Die Gegenkontaktfläche besteht je nach Ausführungsform aus einem elektrischen Kontakt, der mit einer im Gehäuse 1 angeordneten Batterie oder einem Akkumulator verbunden ist oder aus einer Lichtaustrittsöffnung mit einer im Aufsatz 7 angeordneten Lampe, die von der im Gehäuse 1 vorhandenen Batterie gespeist wird.

Zur Verbindung des eigentlichen Nasenspekulums 2 mit dem Handgriff wird die Aussparung 61 über den Achsstift 71 geschoben, wobei die Aussparung 61 schräg von der Stirnfläche des Kontaktfußes 6 zur Mitte hin verläuft, so daß ein schräges Aufstecken des Kontaktfußes 6 auf den gabelförmigen Aufsatz 7 möglich ist. Durch Kippen des Kontaktfußes 6 um den Achsstift 71 werden die obere und untere Fläche des Kontaktfußes 6 bzw. des Aufsatzes 7 zueinander bewegt bis die beiden Kontaktflächen sich miteinander berühren, wobei in dieser Stellung die federnden Nocken 62 in die nutförmige Vertiefungen 72 einrasten, so daß eine für Manipulationen feste Verbindung zwischen Nasenspekulum 2 und Handgriff bzw. Gehäuse 1 sichergestellt ist.

Durch die Verbindung der beiden Kontaktflächen wird entweder die Spannungsversorgung für eine an der Innenfläche der Branche 3 angeordnete Lampe hergestellt oder eine im Aufsatz 7 befindliche Lampe eingeschaltet, so daß deren Licht über einen Lichtleiter zu der distalen Lichtaustrittsoptik an der Innenfläche der Branche 3 Übertragen werden kann.

Figur 2 zeigt in teilweise geschnittener Seitenansicht ein Nasenspekulum mit einer in dem stirnseitigen Aufsatz angeordneten Lichtquelle und einer Lichtleitung 9, die zu einer distalen Lichtaustrittsoptik in der mit dem Kontaktfuß 6 verbundenen Branche 3' führt. Wie insbesondere die vergrößerte Darstellung der Branche 3' im Querschnitt gemäß Figur 3 zeigt, ist die Lichtleitung 9 in einem Kanal 33 im Innern der Branche 3' verlegt und führt zu einer distalen Lichtaustrittsfläche 32 der Lichtleitung 9, die so geformt ist, daß das über die Lichtleitung 9 geführte Licht möglichst gut auf das zu betrachtende Objekt fällt.

Die Lichtleitung 9 führt zu einer optischen Kontaktfläche 90 an der unteren Stirnfläche des Kontaktfußes 6 und korrespondiert dort mit einer entsprechenden Lichtaustrittsfläche an der oberen Stirnfläche des gabelförmigen Aufsatzes 7. Die Lichtquelle besteht in dem dargestellten Ausführungsbeispiel aus einer Lampe 11, die in einer axial beweglichen Fassung 12 gehalten ist, so daß bei Aufstecken und Niederdrücken des Kontaktfußes 6 auf bzw. in den gabelförmigen Aufsatz 7 die Fassung 12 mit der darin befindlichen Lampe 11 nach unten gedrückt wird, so daß der Fußkontakt der Lampe 11 bei aufgesetztem Kontaktfuß 6 einen Pol der als Spannungsquelle dienenden Batterie 10 berührt, während die Fassung 12 mit dem anderen Pol der Batterie 10 verbunden ist, wobei diese Verbindung vorzugsweise durch das Gehäuse 1 hergestellt wird. Am unteren Ende des Gehäuses 1 ist eine Schraubkappe 14 vorgesehen, über die die Batterie oder der Akkumulator 10 in das Gehäuse 1 einsetzbar ist.

Die bewegliche Branche 4' ist über ein Gelenk 20 mit der fest mit dem Gehäuse 1 verbundenen Branche 3' drehbar verbunden, wobei die Ruhestellung durch eine Blattfeder 15' hergestellt wird, die beim hier dargestellten Ausführungsbeispiel zwischen feststehender und beweglicher Branche 3 bzw. 4' vorgesehen und mit der erstgenannten fest verbunden ist.

Die Glühlampe 11, die aus einer Halogenlampe oder dgl. bestehen kann, wird von der Batterie 10 gespeist und das von ihr abgegebene Licht über die Lichtleitung 9 zu der distalen Lichtaustrittsoptik der mit dem Kontaktfuß 6 verbundenen Branche 3' geleitet. Der mit der beweglichen Branche 4 verbundene Griffhebel 5 kann wahlweise mit dem linken Daumen oder bei weiterem Umgreifen des Gehäuses 1 mit dem linken Zeigefinger bedient werden, so daß die Branchen 3, 4 auseinanderspreizen und einen Durchblickbereich ausbilden, durch den der behandelnde Arzt die betreffende Körperhöhle eines Patienten einsehen oder einen Tubus einführen kann. Durch das von der Lichtaustrittsoptik abgegebene Licht wird die zu betrachtende Körperhöhle optimal ausgeleuchtet und ermöglicht dem behandelnden Arzt eine Einsichtnahme ohne störende Schatten oder die Notwendigkeit bestimmter Blickwinkel.

Die jeweilige Spreizstellung der Branchen 3', 4' kann mittels der Rändelschraube 30' eingestellt und fixiert werden. Diese Rändelschraube ist auf einer um eine Drehachse 37 schwenkbaren Gewindestange 35 drehbar. Die Drehachse 37 ist parallel zur Schwenkachse 20' der Branche 4' gerichtet. Durch die schwankbare Ausführung besteht der Vorteil, daß die beiden Achsen einander relativ dicht benachbart angeordnet sein können, ohne daß der Durchlaß für die Gewindestange 35 im Griffteil 5' geschlitzt sein müßte. Mit der Rändelschraube ist eine feinfühlige Einstellung möglich.

Bei dem in Figur 2 dargestellten Ausführungsbeispiel ist die durch die Rändelschraube 30' begrenzte Endstellung der Branche 4' (geschlossene Stellung der Branchen) durchgezogen wiedergegeben. Beim Anziehen der Rändelschraube 30' in Richtung Drehachse 37 wird die Branche in einer geöffneten Position gehalten. Aus der mit der Rändelschraube 30' definierten Anschlagposition kann die bewegliche Branche über den Hebel 5' durch Daumen- oder Handflächendruck jeweils in eine weit geöffnete Stellung gebracht werden, wobei die Position mit der weitesten Öffnung in Figur 2 gestrichelt dargestellt ist. In dieser Position ist die bewegliche Branche mit 4" und der Griffteil mit 5" bezeichnet. Es ist ersichtlich, daß der Griffteil 5" der benachbarten Kontur der entsprechenden Seitenlinie des Batterieteils 1 angepaßt ist. Bei dieser Ausgestaltung ergibt sich eine ergonomische Handhabung, weil Batterieteil und Betätigungselemente günstig in der Hand liegen und die Öffnung der Branchen des Nasenspekulums über den gesamten Öffnungsweg feinfühlig dosierbar ist.

In den Figuren 3 bis 3b ist der Aufsatz gemäß Figur 2 in vergrößerter Form detailliert dargestellt, wobei auf der Gewindeachse 35 eine Begrenzung 36 für die Rändelschraube 30' erkennbar ist, die ein unbeabsichtigtes Abdrehen der Rändelschraube von der Gewindestange 35 verhindert, so daß diese nicht verloren werden kann. Die Endstellung der Rändelschraube 30 entspricht der geschlossenen Stellung der Branchen.

Bei der in Figur 3 dargestellten Variante der Branche 3' ist das Ende 8' des Glasfaserbündels 33 erweitert, so daß sich infolge der fächerförmigen Ausrichtung des Lichtstrahls eine größere Beleuchtungsfläche ergibt. In Figur 3b ist der Querschnitt des Faserbündels 33' wiedergegeben, der sich im Querschnitt teilringförmig der konkaven Innenfläche der Branche 3' anpaßt, so daß der Querschnitt des Faserbündels die Sichtfläche möglichst wenig beeinträchtigt und ein Übergang in den aufgefächerten Bereich 8' in Figur 3 ohne Biegungen mit engen Radien möglich ist.

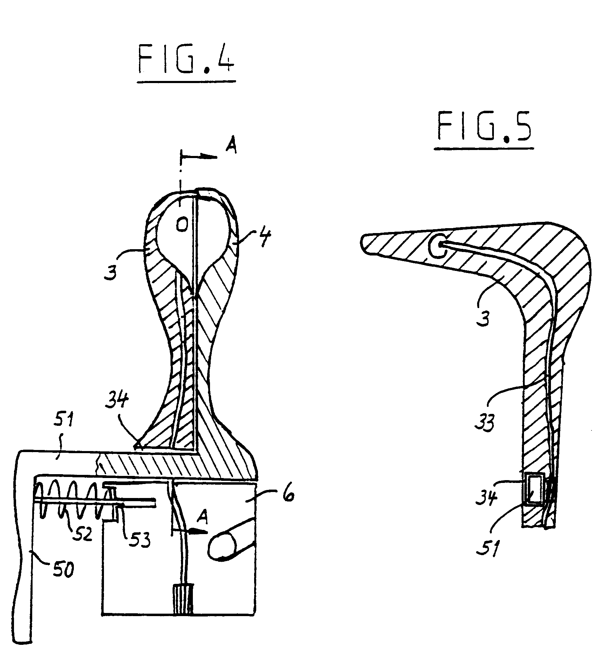

In den Figuren 4 und 5 ist eine Variante des Spreizmechanismus der beiden Branchen 3, 4 im Querschnit bzw. Längsschnitt durch eine der Branchen dargestellt.

In diesem Ausführungsbeispiel ist die Branche 3 fest mit dem Kontaktfuß 6 verbunden und enthält einen Kanal zur Aufnahme einer elektrischen Leitung oder einer Lichtleitung je nach Ausführung der am distalen Ende vorgesehenen Beleuchtungseinrichtung. Zusätzlich ist ein Kanal 34 oberhalb des Kontaktfußes 6 vorgesehen, der zur Aufnahme einer Schubstange 51 dient, die mit der beweglichen Branche 4 verbunden ist. An ihrem anderen Ende ist die Schubstange 51 mit einem abgewinkelten Griffhebel 50 versehen, der gegen die Rückstellwirkung einer Schraubenfeder 52 in Richtung auf das Gehäuse 1 bewegbar ist und ein Auseinanderspreizen der Branchen 3, 4 bewirkt. Vorzugsweise ist die Schraubenfeder 52 um eine Achse 53 angeordnet, die entweder am Griffhebel 50 oder am Kontaktfuß 6 befestigt ist und durch eine Bohrung im Griffhebel 50 oder im Kontaktfuß 6 geführt wird.

Der Führungskanal 34 kann im Querschnitt rechteckförmig oder quadratisch ausgebildet sein und die Schubstange 51 einen entsprechenden Querschnitt aufweisen, so daß eine exakte Führung der beweglichen Branche 4 sichergestellt ist. Zusätzlich kann auf der oberen Stirnseite des Kontaktfußes 6 eine Führungsnut, vorzugsweise als Schwalbenschwanzführung ausgebildet, vorgesehen sein, die eine zusätzliche Führung der beweglichen Branche 4 ermöglicht. Anstelle eines rechteckförmigen oder quadratischen Führungskanals mit entsprechend angepaßter Schubstange können auch zwei nebeneinanderliegende Bohrungen mit zwei im Querschnitt angepaßten Schubstangen vorgesehen werden, wobei der Leitungskanal für die elektrische Leitung oder den Lichtleiter zwischen beiden Bohrungen zum distalen Ende der Branche 3 führt.

Zusätzlich kann bei den in den Figuren dargestellten Ausführungsformen eine Lichtübertragung auch zur beweglichen Branche 4 über das Gelenk 20 erfolgen, wobei analog zur Lichtleitung 9 in der Branche 3 eine Lichtleitung in der beweglichen Branche 4 verlegt ist und das Licht über eine entsprechende Optik im Gelenk 20 in die Lichtleitung der beweglichen Branche 4 eingekoppelt wird.

Es ist ersichtlich, daß bei den in den Figuren dargestellten Ausführungsbeispielen vom die Energiequellen beinhaltenden Gehäuse in Richtung der Branchen keine Teile oder Bauelemente hervorragen, welche die Bewegungsmöglichkeiten bei der Anwendung am Patienten behindern. Insbesondere sind die Betätigungselemente seitlich angebracht, so daß der auftretende Bedienungshub ebenfalls nicht zu Lasten der Bewegungsfreiheit geht. Die Hand des Bedienenden, die das Instrument hält ist die einzige Begrenzung für den Bewegungsbereich am Patienten.

## Patentansprüche

1. Nasenspekulum mit zwei gegen die Wirkung einer Rückstellfeder mit einem Griffhebel auseinanderbewegbaren schnabelförmigen Branchen bei dem an der Innenfläche mindestens einer der Branchen (3, 4) eine distale Beleuchtungseinrichtung (8) vorgesehen ist, die über einen Lichtleiter (9) und eine Lichtquelle (11) an eine Spannungsquelle (10) in einem Gehäuse (1), enthaltend eine Batterie oder einen Akkumulator, anschließbar ist, das Gehäuse (1) einen stirnseitigen Aufsatz (7) aufweist, in den ein Kontaktfuß (6) der mit der distalen Beleuchtungseinrichtung (8) versehenen Branche (3) abnehmbar einrastbar ist und die andere Branche (4) mittels des dem Kontaktfuß (6) benachbarten gegen die Wirkung der Rückstellfeder (15, 52) beweglichen Griffhebels (5, 50) relativ zur anderen Branche (3) beweglich ist, wobei der Griffhebel (5) seitlich des Gehäuses (1) quer zur Richtung der schnabelförmigen Branchen angeordnet und zum Öffnen der Branchen in Richtung auf das Gehäuse (1) verschieb- oder verschwenkbar ist.

2. Nasenspekulum nach einem der vorangehenden Ansprüche, **dadurch gekennzeichnet,** daß das Instrument im Bereich des Gehäuses (1) keine eine den Griffhebel (5) umfassende Hand in Richtung der Branchen überragenden Teile aufweist.

3. Nasenspekulum nach einem der vorangehenden Ansprüche, **dadurch gekennzeichnet,** daß der Betätigungsgriff bei extremer Betätigung an dem Gehäuse (1) anliegt und/oder sich im wesentlichen parallel zu dessen Außenkontur erstreckt.

4. Nasenspekulum nach Anspruch 2 , **dadurch gekennzeichnet,** daß die bewegliche Branche (4) mittels eines Gelenkes (20) mit der den Kontaktfuß (6) aufweisenden Branche (3) verbunden ist und daß der mit der beweglichen Branche (4) verbundene Griffhebel (5) mittels der zwischen Griffhebel (5) und Kontaktfuß (6) angeordneten Rückstellfeder (15) in der Ruhestellung an der den Kontaktfuß (6) aufweisenden Branche (3) anliegt.

5. Nasenspekulum nach Anspruch 2 , **dadurch gekennzeichnet,** daß die bewegliche Branche (4) mit einer Schubstange (51) verbunden ist, die durch einen oberhalb des Kontaktfußes (6) angeordneten Kanal (34) in einem die Branche (3) mit dem Kontaktfuß (6) verbindenden Teil (31) geführt ist und daß die im Querschnitt an den Kanalquerschnitt angepaßte Schubstange (51) an ihrem freien Ende einen Griffhebel (50) aufweist, zwischen dem und dem Kontaktfuß (6) eine Rückstellfeder (52) angeordnet ist.

6. Nasenspekulum nach einem der vorangehenden Ansprüche, **dadurch gekennzeichnet,** daß der stirnseitige Aufsatz (7) einen Achsstift (71) aufweist, der in eine im Kontaktfuß (6) vorgesehene Aussparung (61) einschiebbar ist, und daß in den seitenflächen des Kontaktfußes (6) Nocken (62) vorgesehen sind, die zur festen Verbindung zwischen Kontaktfuß (6) und stirnseitigem Aufsatz (7) in entsprechend angeordnete Nuten (72) an den Seitenflächen (73, 74) des gabelförmigen Aufsatzes (7) einrasten.

7. Nasenspekulum nach einem der vorangehenden Ansprüche, **dadurch gekennzeichnet,** daß das Gehäuse (1) als im wesentlichen zylindrischer Handgriff ausgebildet ist, bei dem die Stirnfläche im Bereich einer der zylindrischen Deckflächen angeordnet ist.

8. Nasenspekulum nach Anspruch 6 , **dadurch gekennzeichnet,** daß die Lichtquelle aus einer in einer axial beweglichen, mit einem Pol der Spannungsquelle (10) verbundenen Fassung (12) gehaltenen Lampe (11) besteht und daß der Fußkontakt der Lampe (11) bei aufgesetztem Kontaktfuß (6) einen mit dem anderen Pol der Spannungsquelle (10) verbundenen Kontaktstift (13) berührt.

9. Nasenspekulum nach einem der vorangehenden Ansprüche, **dadurch gekennzeichnet,** daß die Rückstellfeder aus einer Blatt- oder Schraubenfeder (15 bzw. 52) besteht.

10. Nasenspekulum nach einem der vorangehenden Ansprüche, **dadurch gekennzeichnet,** daß zwischen der beweglichen Branche (4) und der festen Branche (3) eine einstellbare Rast- oder Anschlagvorrichtung vorgesehen ist.

11. Nasenspekulum nach einem der vorangehenden Ansprüche, **dadurch gekennzeichnet,** daß an der Innenfläche der beweglichen Branche (4) eine zusätzliche distale Beleuchtungseinrichtung vorgesehen ist, die über einen an dem die bewegliche Branche (4) mit der festen Branche (3) verbindenden Gelenk (20) vorgesehenen Gleitkontakt mit der Spannungs- oder Lichtquelle (10, 11) verbunden ist.

12. Nasenspekulum nach einem der vorangehenden Ansprüche, **dadurch gekennzeichnet,** daß der Lichtleiter in seinem Verlauf der konkaven Innenkontur der Branche angepaßt und/oder am Lichtaustrittsende der Lichtleitfaser divergierend aufgefächert ist.

## Claims

1. Nasal speculum with two beak-shaped branches moveable apart against the action of a pull-back spring by means of a hand lever, in which on the inside surface of at least one of the branches (3, 4) a distal illuminating device (8) is provided, which is connectable via a light guide (9) and a light source (11) to a voltage source (10) in a housing (1) containing a battery or an accumulator,
the housing (1) comprises a front-side cap (7) into which a contact foot (6) of the branch (3) provided with the distal illuminating device (8) is removably engageable and
the other branch (4) is moveable relative to the other branch (3) by means of the hand lever (5, 50) adjacent to the contact foot (6) and moveable against the action of the pull-back spring (15, 52),
the hand lever (5) being arranged at the side of the housing (1) obliquely to the direction of the beak-shaped branches and being displaceable or swivelable for the opening of the branches in the direction of the housing (1).

2. Nasal speculum according to any one of the preceding claims, characterised in that the instrument does not exhibit in the area of the housing (1) any parts which project in the direction of the branches above a hand grasping the hand lever (5).

3. Nasal speculum according to any one of the preceding claims, characterised in that the operating lever when at the operating limit rests against the housing (1) and/or extends substantially parallel to the external contour of the latter.

4. Nasal speculum according to claim 2, characterised in that the moveable branch (4) is connected by means of a joint (20) to the branch (3) exhibiting the contact foot 16) and that the hand lever (5) connected to the moveable branch (4) rests in the initial position against the branch (3) exhibiting the contact foot (6) by means of the pull-back spring (15) arranged between hand lever (5) and contact foot (6).

5. Nasal speculum according to claim 2, characterised in that the moveable branch (4) is connected to a push rod (51) which is run through a channel (34) arranged above the contact foot (6) in a part (31) connecting the branch (3) to the contact foot (6) and that the push rod (51) adapted in cross-section to the channel cross-section exhibits at its free end a hand lever (50) between which hand lever (50) and the contact foot (6) a pull-back spring (52) is arranged.

6. Nasal speculum according to any one of the preceding claims, characterised in that the front-side cap (7) comprises an axial pin (71) which is insertable into a recess (61) provided in the contact foot (6), and that in the lateral faces of the contact foot (6) lugs (62) are provided which engage for a firm connection between contact foot (6) and front-side cap (7) in correspondingly arranged grooves (72) on the lateral faces (73, 74) of the forked cap (7).

7. Nasal speculum according to any one of the preceding claims, characterised in that the housing (1) is formed as a substantially cylindrical handle in which the front face is arranged in the area of one of the cylindrical top faces.

8. Nasal speculum according to claim 6, characterised in that the light source consists of a lamp (11) held in an axially moveable holder (12) connected to one pole of the voltage source (10) and that the foot contact of the lamp (11) touches when the contact foot (6) is placed in position a contact pin (13) connected to the other pole of the voltage source (10).

9. Nasal speculum according to any one of the preceding claims, characterised in that the pull-back spring consists of a leaf spring or coil spring (15 or 52).

10. Nasal speculum according to any one of the preceding claims, characterised in that between the moveable branch (4) and the fixed branch (3) an adjustable catching or stop device is arranged.

11. Nasal speculum according to any one of the preceding claims, characterised in that on the inside surface of the moveable branch (4) an additional distal illuminating device is provided, which is connected to the voltage source or light source (10, 11) via a sliding contact provided on the joint (20) connecting the moveable branch (4) to the fixed branch (3).

12. Nasal speculum according to any one of the preceding claims, characterised in that the light guide is adapted in its course to the concave inner contour of the branch and/or fanned out divergently at the light exit end of the optical fibre.

**Revendications**

1. Spéculum nasal comportant deux branches en forme de bec, écartables à l'aide d'un levier à poignée contre l'action d'un ressort de rappel, dans lequel il est prévu sur la face inférieure d'au moins une des branches (3, 4) un dispositif d'éclairage distal (8) raccordable par un câble fibre optique (9) et une source lumineuse (11) à une source de tension (10) dans un boîtier (1) contenant une pile ou un accumulateur ; dans lequel le boîtier (1) présente un embout frontal (7) dans lequel un sabot de contact (6) de la branche (3) munie du dispositif d'éclairage distat (8) est encastrable de façon amovible et dans lequel l'autre branche (4) est mobile par rapport à l'autre branche (3) au moyen du levier à poignée (5, 50) voisin du sabot de contact (6), mobile contre l'action du ressort de rappel (15, 52), te levier à poignée (5) étant disposé latéralement par rapport au boîtier (1), perpendiculairement à ta direction des branches en forme de bec et étant déplaçable ou basculable en direction du boîtier (1) en vue de l'ouverture des branches.

2. Spéculum nasal selon la revendication précédente, caractérisé en ce que l'instrument ne présente au niveau du boîtier (1) aucune pièce surplombant la main tenant le levier à poignée (5) en direction des branches.

3. Spéculum nasal selon l'une des revendications précédentes, caractérisé en ce que la poignée de manoeuvre, pour une manoeuvre extrême, repose contre le boîtier (1) et/ou s'étend sensiblement parallèlement à son contour externe.

4. Spéculum nasal selon la revendication 2, caractérisé en ce que la branche mobile (4) est reliée au moyen d'un joint articulé (20) à la branche (3) présentant te sabot de contact (6) et en ce que le levier à poignée (5) relié à la branche mobile (4) repose à sa position de repos contre la branche (3) présentant le sabot de contact (6) grâce au ressort de rappel (15) disposé entre le levier à poignée (5) et le sabot de contact (6).

5. Spéculum nasal selon la revendication 2, caractérisé en ce que la branche mobile (4) est reliée à un levier de commande (51) qui est guidé par un canal (34) disposé au-dessus du sabot de contact (6) dans une pièce (31) reliant la branche (3) au sabot de contact (6) et en ce que te levier de commande (51) dont la section transversale est adaptée à la section transversale du canal présente à son extrémité libre un levier à poignée (50) entre lequel et le sabot de contact (6) est disposé un ressort de rappel (52).

**6.** Spéculum nasal selon l'une des revendications précédentes, caractérisé en ce que l'embout frontat (7) présente une goupille axiale (71) insérable dans un évidement (61) ménagé dans le sabot de contact (6) et en ce que sont prévus dans tes faces latérales du sabot de contact (6) des ergots (62) qui, en vue d'un assemblage solide entre le sabot de contact (6) et l'embout frontal (7) s'engagent dans des rainures (72) correspondantes ménagées dans les faces latérales (73, 74) de l'embout (7) bifurqué.

**7.** Spéculum nasal selon l'une des revendications précédentes, caractérisé en ce que le boîtier (1) a sensiblement le forme d'un manche cylindrique dont la surface frontale est disposée au niveau d'une des surfaces de recouvrement cylindriques.

**8.** Spéculum nasal selon ta revendication 6, caractérisé en ce que la source lumineuse est composée d'une lampe (11) maintenue dans une douille (12) mobile axialement, reliée à un pôle de la source de tension (10) et en ce que le contact du pied de la lampe (11) touche, lorsque te sabot de contact (6) est mis en place, une pointe de contact (13) reliée à l'autre pôle de la source de tension (10).

**9.** Spéculum nasal selon l'une des revendications précédentes, caractérisé en ce que te ressort de rappel est composé d'un ressort à lame ou d'un ressort à boudin (15 ou 52).

**10.** Spéculum nasal selon l'une des revendications précédentes, caractérisé en ce qu'est prévu entre ta branche mobile (4) et la branche fixe (3) un dispositif réglable d'encliquetage ou de butée.

**11.** Spéculum nasal selon l'une des revendications précédentes, caractérisé en ce qu'est prévu, sur la face interne de la branche mobile (4), un dispositif d'éclairage distal additionnel qui est relié à la, source de tension ou à la source lumineuse (10, 11) par un contact à frottement prévu sur le joint articulé (20) reliant la branche mobile (4) à la branche fixe (3).

**12.** Spéculum nasal selon l'une des revendications précédentes, caractérisé en ce que le câble fibre optique est adapté sur son trajet au contour interne concave de la branche et/ou s'évase par divergence à l'extrémité sortie de la lumière de la fibre optique.

# FIG.1

Fig. 2

Fig. 3b

Fig. 3a

Fig. 3

# FIG.4

# FIG.5